# EUROPÄISCHE PATENTSCHRIFT

(11) **EP 3 548 864 B1**
(45) Veröffentlichungstag und Bekanntmachung des Hinweises auf die Patenterteilung: **22.09.2021**
(21) Anmeldenummer: 17808919.9
(22) Anmeldetag: 05.12.2017
(51) Int. Cl.: G01N 1/30, G01N 33/483, G02B 21/34, G01N 27/447

(54) **VERFAHREN ZUR HERSTELLUNG TRANSPARENTER BIOLOGISCHER PRÄPARATE FÜR EINE LICHTMIKROSKOPISCHE UNTERSUCHUNG**
METHOD FOR PREPARING TRANSPARENT BIOLOGICAL SAMPLES FOR LIGHT MICROSCOPY ANALYSIS
PROCÉDÉE DE PRÉPARATION D'ÉCHANTILLONS BIOLOGIQUES TRANSPARENTS POUR ANALYSE PAR MICROSCOPIE OPTIQUE

(30) Priorität: 05.12.2016 DE 102016123458
(43) Veröffentlichungstag der Anmeldung: 09.10.2019
(73) Patentinhaber: Georg-August-Universität Göttingen Stiftung Öffentlichen Rechts, Universitätsmedizin, 37075 Göttingen (DE)
(72) Erfinder: VENTZKI, Robert, 69221 Dossenheim (DE); WOUTERS-BUNT, Fred S., 37085 Göttingen (DE)
(74) Vertreter: REHBERG HÜPPE + PARTNER
(86) Internationale Anmeldenummer: PCT/EP2017/081476
(87) Internationale Veröffentlichungsnummer: WO 2018/104282

(56) Entgegenhaltungen:
- WO-A1-2015/041755
- WO-A1-2016/108359
- US-A1- 2015 144 490
- SUNG-YON KIM ET AL: "Stochastic electrotransport selectively enhances the transport of highly electromobile molecules", PROCEEDINGS NATIONAL ACADEMY OF SCIENCES PNAS, Bd. 112, Nr. 46, 2. November 2015 (2015-11-02), Seiten E6274-E6283, XP055434816, US ISSN: 0027-8424, DOI: 10.1073/pnas.1510133112

## Beschreibung

### TECHNISCHES GEBIET DER ERFINDUNG

Die Erfindung bezieht sich auf ein Verfahren zur Herstellung transparenter biologischer Präparate für eine lichtmikroskopische Untersuchung. Insbesondere bezieht sich die vorliegende Erfindung auf ein solches Verfahren, bei dem biologisches Gewebe elektrophoretisch geklärt wird, indem das Gewebe in eine wässrige alkalische Elektrophoreselösung eingetaucht und in der Elektrophoreselösung einem elektrischen Feld ausgesetzt wird, wobei die Elektrophoreselösung eine Base und ein Detergens enthält.

Transparente biologische Präparate sind notwendig, um die Präparate zum Beispiel mittels Lichtblattmikroskopie dreidimensional abbilden zu können. Um die Transparenz von biologischen Präparaten zu erreichen, sind insbesondere Häm-Gruppen des Blutfarbstoffs Hämoglobin und Lipide aus den biologischen Präparaten zu entfernen.

### STAND DER TECHNIK

Aus der US 2015/0144490 A1 ist ein auch als CLARITY-Verfahren bekanntes Vorgehen beim Präparieren biologischer Proben für die mikroskopische Analyse bekannt. Beim CLARITY-Verfahren wird die jeweilige Probe zunächst in Hydrogel fixiert. Erst danach wird die Probe in eine wässrige alkalische Elektrophoreselösung gegeben und in dieser Elektrophoreselösung einem elektrischen Feld ausgesetzt. Die Elektrophoreselösung enthält Borsäure, 0,4 % (m/v) Natriumdodecylsulfat (SDS) und Natriumhydroxid (NaOH) zur Einstellung eines pH-Werts von 8,5. Die Elektrophoreselösung wird in der Kammer, in der die jeweilige Probe dem elektrischen Feld ausgesetzt wird, umgewälzt. Die Temperatur der Elektrophoreselösung beträgt dabei 37-50 °C, die angelegte elektrische Spannung 10-60 V (ohne eine Angabe der Strecke, über der diese Spannung abfällt). Praktisch benötigt die elektrophoretische Klärung nach dem CLARITY-Verfahren einige Tage. Nach dem elektrophoretischen Klären wird die Probe in ein Medium mit einem Brechungsindex von 1,5 überführt und sie kann zusätzlich angefärbt werden, beispielweise mittels Antikörperfärbung.

Die US 2005/0130317 A1 beschreibt eine Vorrichtung zur parallelen Analyse von biologischen Molekülen mit einem Reaktionsraum, der sich zwischen zwei Elektroden erstreckt. An die Elektroden wird eine Spannung angelegt, die zu einem Wandern der zwischen die Elektroden eingebrachten biologischen Moleküle führt.

Aus der erst nach dem Prioritätstag dieser Patentanmeldung veröffentlichten WO 2017/096248 A1 sind Verfahren zum Präparieren und Analysieren von Tumorgewebeproben zum Detektieren und Überwachen von Tumoren bekannt. Die Verfahren weisen das Behandeln einer biologischen Probe durch Fixieren der Probe in der Anwesenheit von Hydrogeluntereinheiten, das Polymerisieren der Hydrogeluntereinheiten zum Ausbilden einer in Hydrogel eingebetteten Probe, das Klären der in Hydrogel eingebetteten Probe und das Markieren der geklärten in Hydrogel eingebetteten Probe mit einem oder mehreren detektierbaren Markern auf. Das Klären der Probe umfasst beispielsweise das Aussetzen gegenüber organischen Lösungsmitteln, das Aussetzen gegenüber Detergenzien, wie beispielsweise Saponin, Triton X-100 und Tween-20, das Aussetzen gegenüber ionischen oberflächenaktiven Mitteln, zum Beispiel Natriumdodecylsulfat (SDS), und insbesondere das Elektrophoresieren unter Verwendung einer Pufferlösung, die ein ionisches oberflächenaktives Mittel, insbesondere Natriumdodecylsulfat, aufweist.

### AUFGABE DER ERFINDUNG

Der Erfindung liegt die Aufgabe zugrunde, ein Verfahren zur Herstellung transparenter biologischer Präparate für eine lichtmikroskopische Untersuchung aufzuzeigen, das weniger aufwändig und erheblich schneller ist als das bekannte CLARITY-Verfahren.

### LÖSUNG

Die Aufgabe der Erfindung wird durch ein Verfahren mit den Merkmalen des unabhängigen Patentanspruchs 1 gelöst. Die abhängigen Patentansprüche betreffen bevorzugte Ausführungsformen des erfindungsgemäßen Verfahrens.

### BESCHREIBUNG DER ERFINDUNG

Bei einem erfindungsgemäßen Verfahren zur Herstellung transparenter biologischer Präparate für eine lichtmikroskopische Untersuchung wird biologisches Gewebe elektrophoretisch geklärt, indem das Gewebe in eine wässrige alkalische Elektrophoreselösung eingetaucht und in der Elektrophoreselösung einem elektrischen Feld ausgesetzt wird. Die Elektrophoreselösung enthält eine Pufferbase, deren Kationen ein Molekulargewicht von mindestens 50 Da aufweisen, in einer Konzentration von 5 bis 100 mol/m³ (5 bis 100 mmol/l) und ein nichtionisches Detergens in einer Konzentration von 0,1 bis 10 % (w/v, das heißt Gewicht in Kilogramm bezogen auf das Volumen in Liter).

Mit der Pufferbase, deren Kationen ein Molekulargewicht von mindestens 50 Da aufweisen, stellt die Pufferbase zwar die gewünschte Alkalizität der Elektrophoreselösung ein, ihre Kationen sind aber - insbesondere verglichen mit Natriumionen - vergleichsweise groß und unbeweglich. Dies hat zur Folge, dass das elektrische Feld nicht vornehmlich nur einen elektrischen Strom in Form bewegter Kationen der Pufferbase hervorruft, sondern zu einem wesentlichen Anteil auch einen Strom aus Mizellen, in die Häm-Gruppen und/oder Lipide in das Detergens eingeschlossen sind. Der auf diesen Mizellen basierende elektrische Strom führt zu der gewünschten Klärung des biologischen Gewebes, indem er die Mizellen und damit die Häm-Gruppen und Lipide aus dem biologischen Gewebe herausleitet.

Indem bei dem erfindungsgemäßen Verfahren ein nichtionisches Detergens zur Anwendung kommt, tritt auch kein durch das angelegte elektrische Feld hervorgerufener elektrischer Strom in Form von Detergens-Ionen auf, der als solcher ebenso wie ein Strom der Kationen der Pufferbase in Bezug auf die angestrebte Klärung des biologischen Gewebes nur parasitär wäre. Die Folge solcher parasitärer lonenströme wäre unter anderem eine Erwärmung des biologischen Gewebes, ohne dass diese Erwärmung mit einer Klärung des biologischen Gewebes verbunden wären. Parasitäre Ströme würden auch verhindern, dass elektrische Felder größerer Feldstärke über das biologische Gewebe hinweg ausgebildet werden können, weil die dadurch hervorgerufenen großen Ströme das biologische Gewebe zu sehr aufheizen würden. Solche elektrischen Felder größerer Feldstärke sind aber für das elektrische Herausleiten auch größerer und entsprechend unbeweglicher Mizellen aus dem biologischen Gewebe vorteilhaft.

Weil bei dem erfindungsgemäßen Verfahren sowohl die Kationen der Pufferbase relativ unbeweglich sind als auch das Detergens nichtionisch ist, werden die parasitären Ströme klein gehalten, so dass die Effizienz der elektrophoretischen Klärung bezogen auf die fließenden Ströme erheblich gesteigert wird. Darüber hinaus ist die Neigung nichtionischer Detergenzien, an Proteine anzubinden, die in dem biologischen Gewebe verbleiben sollen, geringer als die Neigung ionischer Detergenzien, wie beispielsweise SDS. Aus diesem Grund kann bei dem erfindungsgemäßen Verfahren ohne Weiteres auf eine Fixierung des biologischen Gewebes in Hydrogel verzichtet werden, mit dem bei dem bekannten CLARITY-Verfahren sichergestellt wird, dass die Proteine in dem jeweiligen biologischen Gewebe zurückbleiben. Der Entfall der Notwendigkeit der Fixierung des biologischen Gewebes in Hydrogel hat dabei nicht nur den Vorteil, dass erheblicher Verfahrensaufwand eingespart wird, sondern es wird auch erreicht, dass die Mobilität der Mizellen, die aus dem biologischen Gewebe mit Hilfe des angelegten elektrischen Felds herausgeleitet werden sollen, nicht durch das Hydrogel herabgesetzt ist.

Insgesamt wird mit dem erfindungsgemäßen Verfahren eine Klärung biologischer Gewebe zur Herstellung von biologischen Präparaten für eine lichtmikroskopische Untersuchung regelmäßig schon binnen weniger Stunden erreicht.

Es versteht sich, dass die Angabe "nichtionisches Detergens" nicht so zu verstehen ist, dass hiermit nur ein Detergens mit einer lonizität von null gemeint ist. Vielmehr bezieht sich die Angabe auf alle Detergenzien, die keine ausgeprägte lonizität, das heißt zumindest im Wesentlichen keine lonizität aufweisen. Vorzugsweise wird das nichtionische Detergens zudem danach ausgewählt, dass es eine möglichst geringe Affinität zu Proteinen aufweist. Der Fachmann ist anhand dieser Vorgaben ohne Weiteres in der Lage, geeignete nichtionische Detergenzien zu selektieren.

Praktische Erprobungen des erfindungsgemäßen Verfahrens haben gezeigt, dass zumindest die folgenden Detergenzien als nichtionische Detergenzien geeignet sind: Tween 20, Tween 80, Triton X45, Triton X100, Triton X102, n-octyl-beta-D-Glucopyranosid, Octylphenolethoxylat, Brij35 und Nonidet P40. Besonders gute Eigenschaften zur Verwendung bei der vorliegenden Erfindung zeigten die nichtionischen Detergenzien Tween 80, Triton X45, Triton X102, n-octyl-beta-D-Glucopyranosid, Brij35 und Nonidet P40. Die günstigsten Eigenschaften zeigten Tween 80, Triton X102 und Nonidet P40.

Das nichtionische Detergens, das bei dem erfindungsgemäßen Verfahren zum Einsatz kommt, kann auch aus mehr als einem der voranstehend genannten Detergenzien zusammengesetzt sein. Die Mischung der Detergenzien kann gezielt auf verschiedene beim elektrophoretischen Klären zu entfernenden Inhaltsstoffe des jeweiligen biologischen Gewebes abgestimmt sein.

Die Konzentration der Pufferbase in der Elektrophoreselösung beträgt bevorzugt 10 bis 50 mol/m³ und noch mehr bevorzugt 15 bis 25 mol/m³, das heißt etwa 20 mol/m³.

Die Konzentration des nichtionischen Detergens in der Elektrophoreselösung beträgt vorzugsweise 0,5 bis 1,5 % (w/v), das heißt etwa 1 % (w/v).

Das Molekulargewicht der Kationen der Pufferbase beträgt vorzugsweise mindestens 100 Da. Pufferbasen, die diese Anforderung in der Reihenfolge ihrer Nennung zunehmendem Maße erfüllen, sind Tris, Bicine und BisTris.

Eine Maximaltemperatur der Elektrophoreselösung während des elektrophoretischen Klärens kann in einem Bereich von 20 bis 90 °C gehalten werden. In jedem Fall ist die Temperatur unterhalb eines Siedepunkts der Elektrophoreselösung zu halten. Normalerweise ist es bevorzugt, die Maximaltemperatur der Elektrophoreselösung während des elektrophoretischen Klärens in einem Bereich vom 40 bis 60 °C, das heißt bei ungefähr 50 °C zu halten. Mit einer darüberhinausgehenden Temperatur kann jedoch erreicht werden, dass Anbindungspunkte für Antikörper an Proteine thermisch demaskiert werden, so dass diese Proteine anschließend mit den Antikörpern markiert werden können.

Bei dem erfindungsgemäßen Verfahren resultiert eine Temperaturerhöhung des biologischen Gewebes durch den Eintrag elektrischer Energie, die in Wärme umgewandelt wird. Dies ist grundsätzlich unvermeidbar. Bei dem erfindungsgemäßen Verfahren ist jedoch die erreichte Klärung des biologischen Gewebes bezogen auf die eingetragene elektrische Energie besonders groß. Damit fällt es auch besonders leicht, die Temperatur des biologischen Gewebes auf eine Maximaltemperatur in den genannten Bereichen zu beschränken, ohne dass hierfür beispielsweise eine aktive Kühlung der Elektrophoreselösung notwendig wäre.

Bei der Durchführung des erfindungsgemäßen Verfahrens kann während des elektrophoretischen Klärens ein pH-Wert der Elektrophoreselösung in einem Bereich von 8 bis 9 gehalten werden. Der in Folge des angelegten elektrischen Felds fließende elektrische Strom reduziert typischerweise den pH-Wert der Elektrophoreselösung, auch wenn die Pufferbase ein Reservoir für OH-Gruppen bereitstellt. Um den Wirkungsgrad der Klärung des biologischen Gewebes hochzuhalten, ist es daher sinnvoll, den pH-Wert der Elektrophoreselösung in den genannten alkalischen Bereich zu halten. Zu diesem Zweck kann während des elektrophoretischen Klärens frische Pufferbase zugesetzt werden. Auch das sich durch das Ausleiten der Mizellen unter Einwirkung des elektrischen Felds verbrauchende Detergens kann während des elektrophoretischen Klärens wieder aufgefüllt werden, indem frisches Detergens zu der Elektrophoreselösung zugesetzt wird. Ein maximaler Wirkungsgrad des erfindungsgemäßen Verfahrens wird erreicht, wenn die Elektrophoreselösung fortlaufend gegen unverbrauchte Elektrophoreselösung ausgetauscht wird.

Bei dem erfindungsgemäßen Verfahren kann eine elektrische Leistung, die an die Elektrophoreselösung und das darin eintauchende Gewebe abgegeben wird, während des elektrophoretischen Klärens geregelt werden. Diese Regelung kann abhängig von der Temperatur des biologischen Gewebes bzw. der Elektrophoreselösung erfolgen oder auch auf einen konstanten Wert, der das Einhalten einer bestimmtem Maximaltemperatur sicherstellt. Zumindest kann die elektrische Leistung für einen Teilzeitraum des elektrophoretischen Klärens auf einen solchen festen Wert geregelt werden. Wenn die Klärung des biologischen Gewebes weit fortgeschritten und/oder die Elektrophoreselösung bereits zu wesentlichen Teilen verbraucht ist, ist das weitere Einregeln der elektrischen Leistung auf einen festen Wert häufig nicht mehr sinnvoll.

Der Fortschritt des Klärens des biologischen Gewebes kann durch Erfassen eines elektrischen Widerstands der Elektrophoreselösung und des darin eingetauchten biologischen Gewebes beobachtet werden. Zunächst nimmt der elektrische Widerstand ab, das heißt die elektrische Leitfähigkeit zu, wenn sich die auszuleitenden Mizellen in dem biologischen Gewebe bilden. Mit dem Verbrauch der Elektrophoreselösung und/oder der bereits erfolgten Ableitung der wesentlichen Teile der zu entfernenden Häm-Gruppen und Lipide nimmt dann der Widerstand wieder zu beziehungsweise die elektrische Leitfähigkeit wieder ab. So können dann bei dem erfindungsgemäßen Verfahren Bedingungen der elektrophoretischen Klärung geändert werden und/oder das elektrophoretische Klären kann beendet werden, wenn der elektrische Widerstand oder seine zeitliche Änderung einen vorgegebenen Grenzwert über- und/oder unterschreitet.

Bevor das biologische Gewebe gemäß dem erfindungsgemäßen Verfahren elektrophoretisch geklärt wird, kann es bereits anderen Behandlungen unterworfen werden. Hierzu zählt, dass das biologische Gewebe fixiert wird, beispielsweise mit Formaldehyd, das die Mobilität der Mizellen anders als ein zur Fixierung verwendetes Hydrogel jedoch nicht einschränkt. Weiterhin zählt das Waschen des biologischen Gewebes, beispielweise mit Wasser oder auch mit der später während des elektrophoretischen Klärens eingesetzten Elektrophoreselösung, zu den möglichen Schritten vor der eigentlichen elektrophoretischen Klärung. Weiterhin kann das biologische Gewebe, bevor es elektrophoretisch geklärt wird, in einer wässrigen alkalischen Lösung inkubiert werden. Bei diesem Inkubieren erweist es sich als günstig, wenn folgende Parameter zumindest teilweise eingehalten werden: Das Inkubieren erfolgt für einen Zeitraum von 30 bis 120 min, vorzugsweise von 45 bis 90 min. Das Inkubieren erfolgt bei einer Temperatur von 20 bis 50 °C, vorzugsweise von 35 bis 40 °C, das heißt von etwa 37,5 °C. Die wässrige alkalische Lösung weist eine Alkalikonzentration von 50 bis 2.000 mol/m³, vorzugsweise von 100 bis 1.000 mol/m³ auf. Die wässrige alkalische Lösung weist NaOH zur Bereitstellung ihrer Alkalikonzentration auf. Die wässrige alkalische Lösung weist einen C₁₋₆-Alkohol in einer Konzentration von 10 bis 70 % (v/v) oder vorzugsweise von 40 bis 60 % (v/v) auf. Die wässrige alkalische Lösung weist Ethanol auf; und die wässrige alkalische Lösung weist ein Detergens in einer Konzentration von 0,1 bis 10 % (w/v) und vorzugsweise von 0,5 bis 2 % (w/v) auf.

Zusätzlich zu der elektrophoretischen Klärung in der alkalischen wässrigen Elektrophoreselösung kann das biologische Gewebe in einer wässrigen sauren Lösung inkubiert und dann in einer wässrigen sauren Elektrophoreselösung zur weiteren elektrophoretischen Klärung einem elektrischen Feld ausgesetzt werden.

Beim Inkubieren des Gewebes in der wässrigen sauren Lösung kann mindestens einer der folgenden Parameter eingehalten werden: Das Inkubieren erfolgt für 30 bis 120 min, vorzugsweise für 45 bis 90 min. Das Inkubieren erfolgt bis 20 bis 50 °C, vorzugsweise bei 35 bis 40 °C. Die wässrige saure Lösung weist eine Protonenkonzentration von 50 bis 2.000 mol/m³, vorzugsweise von 100 bis 1.000 mol/m³ auf. Die wässrige saure Lösung weist Trichlor-Essigsäure auf, um ihre Protonenkonzentration bereitzustellen. Die wässrige saure Lösung weist einen C₁₋₆-Alkohol in einer Konzentration von 10 bis 70 % (v/v), vorzugsweise von 40 bis 60 % (v/v) auf. Die wässrige saure Lösung weist Ethanol auf; und die wässrige saure Lösung weist ein Detergens in einer Konzentration von 0,1 bis 10 % (w/v), vorzugsweise von 0,5 bis 2 % (w/v) auf. Weiterhin kann die wässrige saure Elektrophoreselösung mindestens einen der folgenden Parameter einhalten: Die wässrige saure Elektrophoreselösung enthält eine Puffersäure in einer Konzentration von 5 bis 100 mol/m³ und ein nichtionisches Detergens in einer Konzentration von 0,1 bis 10 % (w/v); und die wässrige saure Elektrophoreselösung enthält Essigsäure in einer Konzentration von 10 bis 50 mol/m³ und ein nichtionisches Detergens in einer Konzentration von 0,5 bis 2 % (w/v).

Nachdem das biologische Gewebe elektrophoretisch geklärt wurde, kann es für die lichtmikroskopische Untersuchung weiter präpariert werden. Hierzu kann mindestens einer der folgenden Schritte zählen: Das biologische Gewebe wird mit mindestens einem Antikörper inkubiert. Das biologische Gewebe wird in einer Lösung eines Antikörpers einem elektrischen Feld ausgesetzt. Das biologische Gewebe wird mit mindestens einem Farbstoff gefärbt. Das biologische Gewebe wird in einer Lösung eines Farbstoffs einem elektrischen Feld ausgesetzt. Das biologische Gewebe wird mit einem organischen Lösungsmittel gewaschen. Das biologische Gewebe wird mit Xylol oder Dichlormethan gewaschen. Das biologische Gewebe wird in eine Lösung mit einem Brechungsindex im Bereich von n = 1,4 bis n = 1,6 eingebracht. Der Brechungsindexbereich von n = 1,4 bis n = 1,6, das heißt von etwa n = 1,5 bedeutet, dass die Lösung denselben Brechungsindex wie das geklärte biologische Gewebe aufweist. Damit treten keine Streuungen an den Grenzflächen des biologischen Gewebes bei seiner lichtmikroskopischen Untersuchung auf.

Konkret kann das biologische Gewebe, nachdem es elektrophoretisch geklärt wurde, in eine wässrige Lösung und/oder in eine Zucker- oder Polyollösung mit einem Brechungsindex in dem genannten Bereich von ungefähr n = 1,5 eingebracht werden. Alternativ kann das biologische Gewebe, nachdem es elektrophoretisch geklärt wurde, in einer Alkoholreihe mit ansteigender Alkoholkonzentration entwässert werden und/oder in ein organisches Lösungsmittel, in Methylsalicylat oder in eine Methylsalicylatlösung mit einem Brechungsindex im Bereich von ungefähr n = 1,5 eingebracht werden.

Zur konkreten Durchführung des erfindungsgemäßen Verfahrens kann das Gewebe für das elektrophoretische Klären in einer Reaktionskammer angeordnet werden, die einen um eine Hochachse rotationssymmetrisch ausgebildeten und eine Taillierung aufweisenden, mit der Elektrophoreselösung aufzufüllenden Reaktionsraum, einen nach unten offenen Ringkanal in den Reaktionsraum unterhalb der Taillierung, der an einen nach oben führenden Gasabführkanal angeschlossen ist, eine erste ringförmige Elektrode innerhalb des Ringkanals und/oder in dem Reaktionsraum unterhalb des Ringkanals und eine zweite ringförmige Elektrode in dem Reaktionsraum oberhalb der Taillierung aufweist. Die erste und die zweite Elektrode werden dann an die beiden Ausgänge einer Gleichspannungsquelle angeschlossen, um das biologische Gewebe dem elektrischen Feld auszusetzten. Das Gewebe wird in einem reduzierten freien Querschnitt des Reaktionsraums in der Taillierung angeordnet, wo sich ein annähernd homogenes elektrisches Feld zwischen den Elektroden konzentriert. Über den Gasabführkanal können an der unteren Elektrode gebildete und von dort aufsteigende Gasblasen abgeleitet werden, so dass sich diese nicht im Reaktionsraum sammeln und den elektrischen Stromfluss behindern. Zudem wird die Entstehung von Knallgas verhindert, wenn die Gasblasen durch Hydrolyse gebildeten Wasserstoff enthalten, der sich mit an der oberen Elektrode durch die Hydrolyse gebildetem Sauerstoff vermischen könnte. Die Reaktionskammer kann weitere Merkmale aufweisen, wie sie aus der US 2005/0130317 A1 bekannt sind.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Detergens die Rede ist, ist dies so zu verstehen, dass genau ein Detergens, zwei Detergenzien oder mehr Detergenzien vorhanden sind. Die in den Patentansprüchen angeführten Merkmale können durch andere Merkmale ergänzt werden oder die einzigen Merkmale sein, die das jeweilige Verfahren oder eine bei dem jeweiligen Verfahren verwendete Lösung aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: ist eine Explosionszeichnung einer Reaktionskammer zur Durchführung des erfindungsgemäßen Verfahrens.
- **Fig. 2**: zeigt ein Detail einer modifizierten Ausführungsform der Reaktionskammer in einem Vertikalschnitt; und
- **Fig. 3**: ist ein Flussdiagramm einer Ausführungsform des erfindungsgemäßen Verfahrens.

### FIGURENBESCHREIBUNG

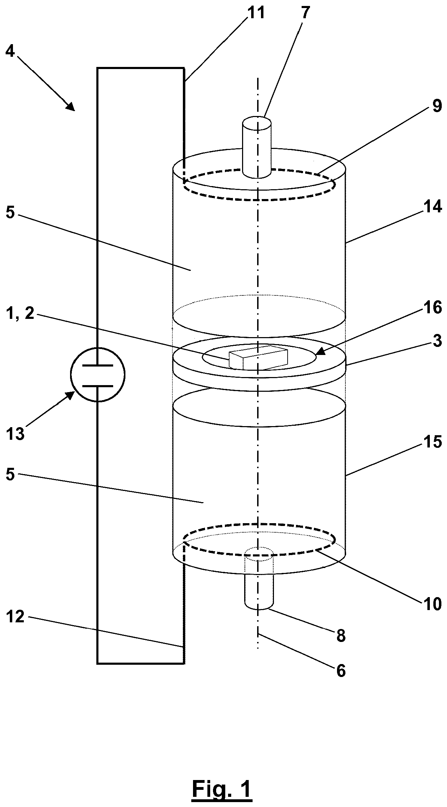

In einer Ausführungsform des erfindungsgemäßen Verfahrens zur Herstellung transparenter biologischer Präparate für eine lichtmikroskopische Untersuchung wird eine Probe 1 aus biologischem Gewebe 2 mit Hilfe eines Probenhalters 3 in einer Reaktionskammer 4 angeordnet, die in **Fig. 1** in einer Explosionszeichnung dargestellt ist. Die Reaktionskammer 4 weist einen Reaktionsraum 5 auf, der rotationssymmetrisch um eine Hochachse 6 ausgebildet ist. In dem Reaktionsraum 5 wird das biologische Gewebe 2 auf etwa halber Höhe angeordnet, wobei durch den Probenhalter 3 eine Taillierung, d. h. eine Durchmesserverringerung des Reaktionsraums 5, ausgebildet ist. Das biologische Gewebe ist dabei in dem Bereich des reduzierten freien Querschnitts 16 des Reaktionsraums 5 in der Taillierung angeordnet. Der Reaktionsraum 5 wird bei der Durchführung des erfindungsgemäßen Verfahrens zumindest mit einer wässrigen alkalischen Elektrophoreselösung befüllt. Auch alle anderen Lösungen, mit denen das biologische Gewebe 2 bei der Durchführung des erfindungsgemäßen Verfahrens in Kontakt kommt, können in den Reaktionsraum 5 eingefüllt beziehungsweise durch den Reaktionsraum 5 hindurchgeleitet werden. Der Reaktionsraum 5 weist hierzu einen oberen Anschluss 7 und einen unteren Anschluss 8 auf. Diese Anschlüsse 7 und 8 können während der Behandlung des biologischen Gewebes 2 nicht nur dazu genutzt werden, die Zusammensetzung der Lösung in dem Reaktionsraum 5 zu ändern oder sogar gegen eine andere Lösung auszutauschen, sondern auch dazu, die jeweilige Lösung durch den Reaktionsraum 5 zu zirkulieren. Bei diesem Zirkulieren kann die jeweilige Lösung zum Beispiel durch einen Wärmetauscher geführt werden, um sie zu temperieren, oder die Lösung kann bezüglich verbrauchter Komponenten regeneriert werden.

Für eine elektrophoretische Klärung des biologischen Gewebes 2, um daraus insbesondere Häm-Gruppen und Lipide zu entfernen, sind eine obere Elektrode 9 und eine untere Elektrode 10 vorgesehen, die in die in dem Reaktionsraum befindliche Lösung eintauchen. Durch Anlegen einer Spannung zwischen den Elektroden 9 und 10 bildet sich ein elektrisches Feld über das biologische Gewebe 2 hinweg aus, das die treibende elektrische Kraft für die elektrophoretische Klärung bereitstellt. Für das Anlegen der Spannung zwischen den Elektroden 9 und 10 sind Anschlussleitungen 11 an die obere Elektrode 9 und 12 an die untere Elektrode vorgesehen, mit denen die Elektroden 9 und 10 an eine Gleichspannungsquelle 13 angeschlossen sind. Die Elektroden 9 und 10 sind ringförmig ausgebildet, um im Bereich der Taillierung, das heißt des freien Querschnitts des Probenhalters 3, in dem das biologische Gewebe 2 angeordnet ist, ein möglichst homogenes elektrisches Feld hervorzurufen. Es versteht sich, dass sowohl der Probenhalter 3 als auch die beiden angrenzenden Teile 14 und 15 der Probenkammer 4 aus elektrisch isolierendem Material ausgebildet sind.

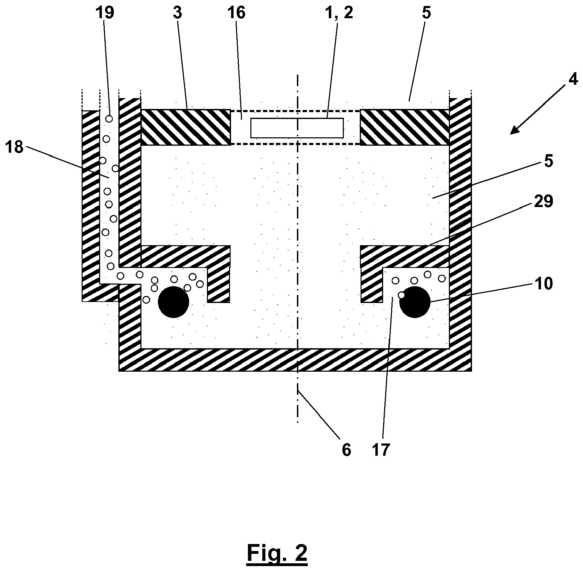

Das in **Fig. 2** in einem Vertikalschnitt dargestellte Detail einer anderen Ausführungsform der Reaktionskammer 4 zeigt den unteren Teil der Reaktionsraumes 5, in den der Probenhalter 3 mit der Probe 1 eingesetzt ist, so dass das biologische Gewebe 2 in dem freien Querschnitt 16 der Taillierung des Reaktionsraums 5 durch den Probenhalter 3 angeordnet ist. Ein unterer Anschluss 8 an den Reaktionsraum 5 ist hier zwar nicht dargestellt, kann aber wie in Fig. 1 vorhanden sein. Die untere Elektrode 10 ist teilweise unterhalb und teilweise innerhalb eines nach oben geschlossenen Ringkanals 17 angeordnet, der durch einen Einsatz 29 in die Probenkammer ausgebildet ist. Der nach unten offene Ringkanal 17 ist an einen nach oben führenden Gasabführkanal 18 angeschlossen, um von der Elektrode 10 in Folge der anliegenden Spannung aufsteigende, beispielsweise durch Hydrolyse gebildeten Wasserstoff enthaltende Gasblasen 19 kontrolliert abzuführen, damit sich diese nicht in dem Reaktionsraum 5 ansammeln und den Stromfluss durch den Reaktionsraum 5 behindern und damit auch die Bildung von Knallgas in dem Reaktionsraum 5 vermieden wird.

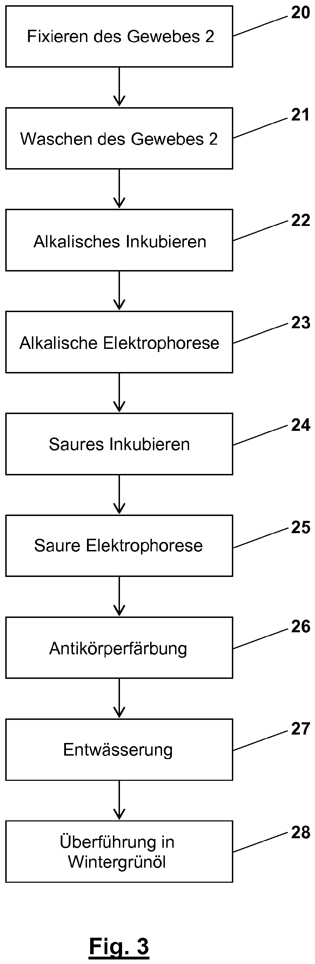

Das in **Fig. 3** in einem Blockdiagramm illustrierte erfindungsgemäße Verfahren beginnt mit einem Fixieren 20 des biologischen Gewebes 2 beispielsweise mit Formaldehyd. Hieran schließt sich ein Waschen 21 des Gewebes 2 beispielsweise mit Wasser an. Im Laufe der Durchführung des erfindungsgemäßen Verfahrens können weitere Waschschritte eingefügt werden, die in Fig. 3 nicht hervorgehoben sind. Grundsätzlich gilt, dass das Gewebe 2 vor jeder längeren Behandlung in einer Lösung, wie sie im Folgenden erläutert werden wird, zunächst mit dieser Lösung gewaschen werden kann.

Ein alkalisches Inkubieren 22 erfolgt zum Beispiel für eine Stunde bei 37 °C in einer wässrigen Lösung mit 100 mol/m³ NaOH, 50 % EtOH und 1 % (w/v) eines Detergens. Eine sich anschließende alkalische Elektrophorese 23 erfolgt beispielsweise für 45 min bei 50 °C in einer wässrigen alkalischen Elektrophoreselösung mit 20 mol/m³Tris und 1 % (w/v) des jeweiligen Detergens. Die eigentliche Elektrophorese erfolgt mit einer Gleichspannung zwischen den Elektroden 9 und 10 von maximal 1000 V, einem maximalen Strom von 150 mA und einer Leistungsbegrenzung auf 10 W. Dies kann z. B. zu einer mittleren Spannung von 500 V bei einem mittleren Strom von 20 mA führen. Hieran kann sich unter Zwischenschaltung eines nicht dargestellten Waschschrittes ein saures Inkubieren 24 anschließen. Das saure Inkubieren 24 kann dem alkalischen Inkubieren 22 entsprechen, außer dass es unter Zusatz von Trichlor-Essigsäure statt NaOH erfolgt. Ebenso können bei einer anschließenden sauren Elektrophorese 25 grundsätzlich dieselben Bedingungen herrschen wie bei der alkalischen Elektrophorese 23, außer dass statt Tris Trichlor-Essigsäure eingesetzt wird. Nachfolgend oder an anderer geeigneter Stelle des erfindungsgemäßen Verfahrens erfolgt eine Antikörperfärbung 26, das heißt eine immunologische Färbung bestimmter Proteine, die nach dem elektrophoretischen Klären in dem Gewebe 2 weiterhin vorhanden sind. Dabei werden Antikörper verwendet, die spezifisch an diese Proteine anbinden und die entweder selbst einen Farbstoff tragen oder nachfolgend mit einem Farbstoff markiert werden. Soweit das geklärte und gegebenenfalls gefärbte biologische Gewebe 2 nicht in einer wässrigen Lösung lichtmikroskopisch untersucht werden soll, erfolgt eine Entwässerung 27, an die sich eine Überführung 28 in ein Medium mit dem Brechungsindex des geklärten Gewebes von etwa 1,5 anschließt. Ein dafür geeignetes Medium ist Wintergrünöl. Anschließend ist das Gewebe zum Beispiel für eine lichtmikroskopische Untersuchung unter Anwendung einer Lichtblatttechnik geeignet. Im Vergleich zu dem bekannten CLARITY-Verfahren zeichnet sich das erfindungsgemäße Verfahren durch eine sehr kurze Gesamtverfahrensdauer von wenigen Stunden aus. Dabei ist auch zu berücksichtigen, dass das saure Inkubieren 24 und die saure Elektrophorese 25 vielfach nicht notwendig sind, um das biologische Gewebe 2 ausreichend zu klären.

Nach dem folgenden Protokoll zur Durchführung des erfindungsgemäßen Verfahrens wurden Proben 1 aus Schweinelungengewebe von jeweils ca. 250 mg erfolgreich geklärt. Das Fixieren 20 des Gewebes 2 erfolgt in Formaldehyd. Das Waschen 21 des Gewebes 2 erfolgt für 10 min mit Wasser. Die alkalische Inkubation 22 erfolgte für eine Stunde bei 37 °C in wässriger Lösung mit 500 mol/m³ NaOH, 50 % (v/v) EtOH und 1 % (w/v) eines nichtionischen Detergens. Die alkalische Elektrophorese 23 erfolgte für 45 min in wässriger Elektrophoreselösung mit 20 mol/m³ Tris und 1 % (w/v) des jeweiligen nichtionischen Detergens. Dabei lag zwischen den Elektroden 9 und 10 eine Gleichspannung von maximal 1000 V und der maximale Strom betrug 150 mA, wobei die Leistung auf 10 W begrenzt wurde. Die Entwässerung 27 erfolgte in vier 30 Minuten-Schritten mit 50 %, 70 %, 90 % und 100 % Ethanol. Das Überführen 28 erfolgte in Wintergrünöl. Die verschiedenen erprobten nichtionischen Detergenzien ergaben bei Anwendung dieses Protokolls unterschiedlich gute Ergebnisse, die wie folgt nach einer Schätzskala von 0 (schlechte Klärung, dunkel-gefärbte Probe) bis hin zu ++++ (gute Durchsichtigkeit und Farblosigkeit) beurteilt wurden:
0: Kein Detergens
+: Tween 20, Triton X100
++: n-Octyl-beta-D-Glucopyranosid, Brie 35
+++: Nonidet P40, Triton X45 (milchige Lösung), Tween 80
++++: Triton X102

In einem weiteren Experiment wurde der Zusammenhang zwischen dem Verlauf des elektrischen Widerstands bei der alkalischen Elektrophorese 23, der Blutbelastung des Gewebes 2 und dem Klärungs-Resultat untersucht. Der elektrische Widerstand sinkt anfangs der alkalischen Elektrophorese 23 ab. Dies ist auf die Bildung und Freisetzung der die Häm-Gruppen und Lipide enthaltenden Mizellen als bewegliche Ladungsträger zurückzuführen, die anschließend durch das elektrische Feld aus dem Gewebe 2 herausgeleitet werden können. Bei hoher Blutbelastung des Gewebes können sich mehr solcher Ladungsträger bilden. Entsprechend nimmt der elektrische Widerstand sehr viel schneller ab und erreicht auch einen kleineren Minimalwert. Mit dem Verbrauch der die Ladungsträger bildenden Komponenten der Elektrophoreselösung steigt der Widerstand wieder an, ebenso wenn die die Ladungsträger bildenden Bestandteile des Gewebes, das heißt die Häm-Gruppen und Lipide, bereits im Wesentlichen aus dem Gewebe entfernt wurden. Dieser Verlauf des elektrischen Widerstands kann gezielt zur Steuerung der alkalischen Elektrophorese genutzt werden, insbesondere um den Zeitpunkt zu erkennen, an dem die alkalische Elektrophorese sinnvollerweise beendet wird. Darüber hinaus ist dieser Verlauf des elektrischen Widerstands ein Beleg für die Richtigkeit der der Erfindung zugrundeliegenden Überlegungen, die elektrische Leitfähigkeit der Elektrophoreselösung selbst möglichst klein zu halten und dafür zu sorgen, dass möglichst nur Ladungsträger gebildet werden, die tatsächlich zu dessen Klärung aus dem Gewebe zu entfernende Substanzen wie Häm-Gruppen und Lipide enthalten. Der unter diesen Bedingungen fließende elektrische Strom führt effizient zur elektrophoretischen Klärung des Gewebes 2. Damit wird sowohl ein elektrisches Aufheizen der Probe 2 durch parasitäre elektrische Ströme vermieden als auch vermieden, dass solche parasitären elektrischen Ströme praktisch das Ausbilden hoher Feldstärken des über die Probe hinweg ausgebildeten elektrischen Felds verhindern, wie sie erforderlich sind, um auf vergleichsweise große Häm-Gruppen und/oder Lipide umfassende Mizellen ausreichend große elektrische Kräfte auszuüben, um sie aus dem biologischen Gewebe 2 herauszuleiten.

### BEZUGSZEICHENLISTE

- 1: Probe
- 2: Biologisches Gewebe
- 3: Probenhalter
- 4: Reaktionskammer
- 5: Reaktionsraum
- 6: Hochachse
- 7: Oberer Anschluss
- 8: Unterer Anschluss
- 9: Obere Elektrode
- 10: Untere Elektrode
- 11: Leitung
- 12: Leitung
- 13: Gleichspannungsquelle
- 14: Oberer Teil der Reaktionskammer 4
- 15: Unterer Teil der Reaktionskammer 4
- 16: Freier Querschnitt
- 17: Ringkanal
- 18: Gasabführkanal
- 19: Gasblasen
- 20: Fixieren
- 21: Waschen
- 22: Alkalisches Inkubieren
- 23: Alkalische Elektrophorese
- 24: Saures Inkubieren
- 25: Saure Elektrophorese
- 26: Antikörperfärbung
- 27: Entwässerung
- 28: Überführung
- 29: Einsatz

## Patentansprüche

1. Verfahren zur Herstellung transparenter biologischer Präparate für eine lichtmikroskopische Untersuchung, wobei biologisches Gewebe (2) elektrophoretisch geklärt wird, indem das Gewebe in eine wässrige alkalische Elektrophoreselösung (23) eingetaucht und in der Elektrophoreselösung einem elektrischen Feld ausgesetzt wird, wobei die Elektrophoreselösung eine Base in einer Konzentration von 5 bis 100 mol/m³ und ein Detergens in einer Konzentration von 0,1 bis 10 % (w/v) enthält, **dadurch gekennzeichnet, dass** die Base eine Pufferbase ist, deren Kationen ein Molekulargewicht von mindestens 50 Da aufweisen, und dass das Detergens ein nichtionisches Detergens ist.

2. Verfahren nach Anspruch 1, **dadurch gekennzeichnet, dass** das nichtionische Detergens zumindest überwiegend aus mindestens einer Substanz zusammengesetzt ist, die aus der Gruppe ausgewählt ist, welche besteht aus: Tween 20, Tween 80, Triton X45, Triton X100, Triton X102, n-octyl-beta-D-Glucopyranosid, Octylphenolethoxylat, Brij35 und Nonidet P40.

3. Verfahren nach einem der vorhergehenden Ansprüche, **dadurch gekennzeichnet, dass** die Elektrophoreselösung die Pufferbase in einer Konzentration von 10 bis 50 mol/m³, bevorzugt von 15 bis 25 mol/m³ enthält.

4. Verfahren nach einem der vorhergehenden Ansprüche, **dadurch gekennzeichnet, dass** die Elektrophoreselösung das nichtionische Detergens in einer Konzentration von 0,5 bis 1,5 % (w/v) enthält.

5. Verfahren nach einem der vorhergehenden Ansprüche, **dadurch gekennzeichnet, dass** das Molekulargewicht der Kationen der Pufferbase mindestens 100 Da beträgt, wobei die Pufferbase optional zumindest überwiegend aus mindestens einer Substanz zusammengesetzt ist, die aus der Gruppe ausgewählt ist, welche besteht aus: Tris, Bicine und BisTris.

6. Verfahren nach einem der vorhergehenden Ansprüche, **dadurch gekennzeichnet, dass** eine Maximaltemperatur der Elektrophoreselösung während des elektrophoretischen Klärens in einem Bereich von 20 bis 90 °C unterhalb eines Siedepunkts der Elektrophoreselösung gehalten wird, wobei die Maximaltemperatur der Elektrophoreselösung während des elektrophoretischen Klärens optional in einem Bereich von 40 bis 60 °C gehalten wird.

7. Verfahren nach einem der vorhergehenden Ansprüche, **dadurch gekennzeichnet, dass** ein pH-Wert der Elektrophoreselösung während des elektrophoretischen Klärens in einem Bereich von 8 bis 9 gehalten wird.

8. Verfahren nach einem der vorhergehenden Ansprüche, **dadurch gekennzeichnet, dass** während des elektrophoretischen Klärens frische Pufferbase und/oder frisches Detergens zu der Elektrophoreselösung zugesetzt oder die Elektrophoreselösung fortlaufend ausgetauscht wird.

9. Verfahren nach einem der vorhergehenden Ansprüche, **dadurch gekennzeichnet, dass** eine elektrische Leistung, die an die Elektrophoreselösung und das darin eingetauchte Gewebe abgegeben wird, während des elektrophoretischen Klärens geregelt wird, wobei die elektrische Leistung optional zumindest für einen Teilzeitraum des elektrophoretischen Klärens auf einen festen Wert geregelt wird.

10. Verfahren nach einem der vorhergehenden Ansprüche, **dadurch gekennzeichnet, dass** während des elektrophoretischen Klärens ein elektrischer Widerstand der Elektrophoreselösung und des darin eingetauchten biologischen Gewebes (2) erfasst wird.

11. Verfahren nach Anspruch 10, **dadurch gekennzeichnet, dass** Bedingungen der elektrophoretischen Klärung geändert werden und/oder das elektrophoretische Klären beendet wird, wenn der elektrische Widerstand oder seine zeitliche Änderung einen vorgegebenen Grenzwert über- und/oder unterschreitet.

12. Verfahren nach einem der vorhergehenden Ansprüche, **dadurch gekennzeichnet, dass** das biologische Gewebe (2), bevor es elektrophoretisch geklärt wird,
- fixiert wird und/oder
- mit Formaldehyd fixiert wird und/oder
- gewaschen wird und/oder
- mit Wasser gewaschen wird und/oder
- mit der Elektrophoreselösung gewaschen wird und/oder
- in einer wässrigen alkalischen Lösung inkubiert wird.

13. Verfahren nach einem der vorhergehenden Ansprüche, **dadurch gekennzeichnet, dass** das biologische Gewebe (2), bevor oder nachdem es in der alkalischen wässrigen Elektrophoreselösung elektrophoretisch geklärt wird/wurde, in einer wässrigen sauren Lösung inkubiert und dann in einer wässrigen sauren Elektrophoreselösung einem elektrischen Feld ausgesetzt wird.

14. Verfahren nach einem der vorhergehenden Ansprüche, **dadurch gekennzeichnet, dass** das biologische Gewebe (2), nachdem es elektrophoretisch geklärt wurde,
- mit einem Antikörper inkubiert wird und/oder
- in einer Lösung eines Antikörpers einem elektrischen Feld ausgesetzt wird und/oder
- mit einem Farbstoff gefärbt wird und/oder
- in einer Lösung eines Farbstoffs einem elektrischen Feld ausgesetzt wird und/oder
- mit einem organischen Lösungsmittel gewaschen wird und/oder
- mit Xylol oder Dichlormethan gewaschen wird und/oder
- in eine Lösung mit einem Brechungsindex im Bereich von n = 1,4 bis n = 1,6 eingebracht wird.

15. Verfahren nach einem der vorhergehenden Ansprüche, **dadurch gekennzeichnet,**
- **dass** das Gewebe für das elektrophoretische Klären in einer Reaktionskammer (4) angeordnet wird, die
- einen um eine Hochachse (6) rotationssymmetrisch ausgebildeten und eine Taillierung aufweisenden, mit der Elektrophoreselösung aufzufüllenden Reaktionsraum (5),
- einen nach unten offenen Ringkanal (17) in dem Reaktionsraum (5) unterhalb der Taillierung, der an einen nach oben führenden Gasabführkanal (18) angeschlossen ist,
- eine erste ringförmige Elektrode (10) innerhalb des Ringkanals (17) und/oder in dem Reaktionsraum (5) unterhalb des Ringkanals (17) und
- eine zweite ringförmige Elektrode (9) in dem Reaktionsraum (5) oberhalb der Taillierung aufweist,
- wobei die erste und die zweite Elektrode (9, 10) an die beiden Ausgänge einer Gleichspannungsquelle (13) angeschlossen werden und
- wobei das Gewebe in einem reduzierten freien Querschnitt (16) des Reaktionsraums (5) in der Taillierung angeordnet wird.

## Claims

1. Method of preparing transparent biological samples for examination by light microscopy, wherein biological tissue (2) is cleared electrophoretically in that the tissue is immersed in an aqueous alkaline electrophoresis solution (23) and subjected to an electric field in the electrophoresis solution, wherein the electrophoresis solution contains a base in a concentration from 5 to 100 mol/m³ and a detergent in a concentration from 0.1 to 10 % (w/v), **characterized in that** the base is a buffer base whose cations have a molecular weight of at least 50 Da, and that the detergent is a non-ionic detergent.

2. Method of claim 1, **characterized in that** the non-ionic detergent at least predominantly consists of at least one substance which is selected from a group consisting of: Tween 20, Tween 80, Triton X45, Triton X100, Triton X102, n-octyl-beta-D-glucopyranoside, octylphenolethoxylate, Brij35 and Nonidet P40.

3. Method of any of the preceding claims, **characterized in that** the electrophoresis solution contains the buffer base in a concentration from 10 to 50 mol/m³, preferably from 15 to 25 mol/m³.

4. Method of any of the preceding claims, **characterized in that** the electrophoresis solution contains the non-ionic detergent in a concentration from 0.5 to 1.5 % (w/v).

5. Method of any of the preceding claims, **characterized in that** the molecular weight of the cations of the buffer base is at least 100 Da, wherein, optionally, the buffer base at least predominantly consists of at least one substance which is selected from a group consisting of: Tris, Bicine and BisTris.

6. Method of any of the preceding claims, **characterized in that** a maximum temperature of the electrophoresis solution is kept in a range from 20 to 90 °C below a boiling point of the electrophoresis solution during the electrophoretic clearing, wherein, optionally, the maximum temperature of the electrophoresis solution is kept in a range from 40 to 60 °C during the electrophoretic clearing.

7. Method of any of the preceding claims, **characterized in that** a pH-value of the electrophoresis solution is kept in a range from 8 to 9 during the electrophoretic clearing.

8. Method of any of the preceding claims, **characterized in that** fresh buffer base and/or fresh detergent is added to the electrophoresis solution or the electrophoresis solution is continuously changed during the electrophoretic clearing.

9. Method of any of the preceding claims, **characterized in that** an electric power which is delivered to the electrophoresis solution and the tissue immersed therein is closed-loop controlled during the electrophoretic clearing, wherein, optionally, the electric power is closed-loop controlled to a constant value at least for a part of a duration of the electrophoretic clearing.

10. Method of any of the preceding claims, **characterized in that** an electric resistance of the electrophoresis solution and the biological tissue (2) immersed therein is registered during the electrophoretic clearing.

11. Method of claim 10, **characterized in that** conditions of the electrophoretic clearing are changed and/or the electrophoretic clearing is terminated, when the electric resistance or its time rate of change goes beyond and/or below a predetermined threshold value.

12. Method of any of the preceding claims, **characterized in that** the biological tissue (2), prior to being cleared electrophoretically,
- is fixed and/or
- is fixed with formaldehyde and/or
- is washed and/or
- is washed with water and/or
- is washed with the electrophoresis solution and/or
- is incubated in an aqueous alkaline solution.

13. Method of any of the preceding claims, **characterized in that** the biological tissue (2), prior to or after being cleared electrophoretically in the alkaline aqueous electrophoresis solution, is incubated in an aqueous acidic solution and then subjected to an electric field in an aqueous acidic electrophoresis solution.

14. Method of any of the preceding claims, **characterized in that** the biological tissue (2), after it has been cleared electrophoretically,
- is incubated with an antibody and/or
- is subjected in a solution of an antibody to an electric field and/or
- is stained with a dye and/or
- is subjected to an electric field in a solution of a dye and/or
- is washed with an organic solvent and/or
- is washed with xylol or dichloromethane and/or
- is placed in a solution with a diffractive index in a range from n = 1.4 to n = 1.6.

15. Method of any of the preceding claims, **characterized in**
- **that** the tissue is arranged in a reaction chamber (4) for the electrophoretic clearing, the reaction chamber comprising
- a reaction room (5) to be filled with the electrophoresis solution, which is rotationally symmetric about a vertical axis (6) and has a waist,
- a downwardly open ring channel (17) in the reaction room (5) below the waist which is connected to an upwardly leading gas removal channel (18),
- a first ring-shaped electrode (10) within the ring channel (17) and/or in the reaction room (5) below the ring channel (17), and
- a second ring-shaped electrode (9) in the reaction room (5) above the waist,
- wherein the first and the second electrode (9, 10) are connected to the two outputs of a direct current source (13), and
- wherein the tissue is arranged in a reduced free cross-section (16) of the reaction room (5) in the waist.

## Revendications

1. Procédé de fabrication de préparations biologiques transparentes pour un examen au microscope optique, dans lequel un tissu biologique (2) subit une clarification par électrophorèse, par immersion du tissu dans une solution électrophorétique (23) alcaline aqueuse et exposition dans la solution électrophorétique à un champ électrique, la solution électrophorétique contenant une base selon une concentration de 5 à 100 mol/m³ et un détergent selon une concentration de 0,1 à 10 % (p/v), **caractérisé en ce que** la base est une base tampon, dont les cations présentent une masse moléculaire d'au moins 50 Da, et **en ce que** le détergent est un détergent non ionique.

2. Procédé selon la revendication 1, **caractérisé en ce que** le détergent non ionique est au moins essentiellement constitué d'au moins une substance, qui est choisie dans le groupe consistant en le Tween 20, le Tween 80, le Triton X45, le Triton X100, le Triton X102, le n-octyl-bêta-D-glucopyranoside, un produit d'éthoxylation de l'octylphénol, le Brij35 et le Nonidet P40.

3. Procédé selon l'une des revendications précédentes, **caractérisé en ce que** la solution électrophorétique contient une base tampon selon une concentration de 10 à 50 mol/m³, de préférence de 15 à 25 mol/m³.

4. Procédé selon l'une des revendications précédentes, **caractérisé en ce que** la solution électrophorétique contient le détergent non ionique selon une concentration de 0,5 à 1,5 % (p/v).

5. Procédé selon l'une des revendications précédentes, **caractérisé en ce que** la masse moléculaire des cations de la base tampon est d'au moins 100 Da, la base tampon étant éventuellement au moins essentiellement constituée d'au moins une substance, qui est choisie dans le groupe qui consiste en le Tris, la Bicine et le Bis Tris.

6. Procédé selon l'une des revendications précédentes, **caractérisé en ce qu'**une température maximale de la solution électrophorétique est, pendant la clarification électrophorétique, maintenue dans une plage de 20 à 90 °C en dessous du point d'ébullition de la solution électrophorétique, la température maximale de la solution électrophorétique étant, pendant la clarification électrophorétique, éventuellement maintenue dans une plage de 40 à 60 °C.

7. Procédé selon l'une des revendications précédentes, **caractérisé en ce que** le pH de la solution électrophorétique est, pendant la clarification électrophorétique, maintenu dans une plage de 8 à 9.

8. Procédé selon l'une des revendications précédentes, **caractérisé en ce que,** pendant la clarification électrophorétique, on ajoute à la solution électrophorétique une base tampon fraîche et/ou un détergent frais, ou encore on remplace en continu la solution électrophorétique.

9. Procédé selon l'une des revendications précédentes, **caractérisé en ce qu'**une puissance électrique qui est cédée à la solution électrophorétique et au tissu qui y est immergé subit une régulation pendant la clarification électrophorétique, la puissance électrique étant éventuellement, au moins pendant une période partielle de la clarification électrophorétique, régulée à une valeur fixe.

10. Procédé selon l'une des revendications précédentes, **caractérisé en ce que,** pendant la clarification électrophorétique, on détermine une résistance électrique de la solution électrophorétique et du tissu biologique (2) qui y est immergé.

11. Procédé selon la revendication 10, **caractérisé en ce qu'**on modifie les conditions de la clarification électrophorétique et/ou on met fin à la clarification électrophorétique quand la résistance électrophorétique, et sa variation dans le temps, passent au-dessus et/ou en dessous d'une valeur limite prédéfinie.

12. Procédé selon l'une des revendications précédentes, **caractérisé en ce que** le tissu biologique (2), avant de subir une clarification électrophorétique,
- est fixé et/ou
- est fixé avec du formaldéhyde et/ou
- est lavé et/ou
- est lavé à l'eau et/ou
- est lavé avec la solution électrophorétique et/ou
- est incubé dans une solution alcaline aqueuse.

13. Procédé selon l'une des revendications précédentes, **caractérisé en ce que** le tissu biologique est, avant ou après qu'il subit ou qu'il a subi une électrophorèse dans la solution électrophorétique aqueuse alcaline, incubé dans une solution acide aqueuse, puis est exposé à un champ électrique dans une solution électrophorétique acide aqueuse.

14. Procédé selon l'une des revendications précédentes, **caractérisé en ce que** le tissu biologique (2), après avoir subi une clarification électrophorétique,
- est incubé avec un anticorps et/ou
- est exposé à un champ électrique dans une solution d'un anticorps et/ou
- est coloré à l'aide d'un colorant et/ou
- est exposé à un champ électrique dans une solution du colorant et/ou
- est lavé avec un solvant organique et/ou
- est lavé avec du xylène ou du dichlorométhane et/ou
- est introduit dans une solution présentant un indice de réfraction dans la plage de n = 1,4 à n = 1,6.

15. Procédé selon l'une des revendications précédentes, **caractérisé en ce que**
- le tissu, pour la clarification électrophorétique, est placé dans une chambre de réaction (4), qui comprend
- un espace de réaction (5), ayant une structure symétrique en rotation autour d'un axe vertical (6), et présentant un rétrécissement de son diamètre, destiné à être rempli de la solution électrophorétique,
- une gaine annulaire (17) ouverte vers le bas dans l'espace de réaction (5) en dessous du rétrécissement du diamètre, qui est raccordée à une gaine d'évacuation des gaz (18) dirigée vers le haut,
- une première électrode annulaire (10) à l'intérieur de la gaine annulaire (17) et/ou dans l'espace de réaction (5) en dessous de la gaine annulaire (17) et
- une seconde électrode annulaire (9) dans l'espace de réaction (5) au-dessus du rétrécissement du diamètre,
- la première et la seconde électrode (9, 10) étant raccordées aux deux sorties d'une source de tension continue (13), et
- le tissu étant disposé dans une section transversale libre réduite (16) de l'espace de réaction (5) dans le rétrécissement du diamètre.
